# EUROPEAN PATENT APPLICATION

(11) **EP 4 197 562 A1**
(43) Date of publication of application: **21.06.2023**
(21) Application number: 21862045.8
(22) Date of filing: 25.08.2021
(51) Int. Cl.: A61K 51/12, B01J 19/12

(54) **MRI CONTRAST MEDIUM INCLUDING CARBON-13-CONTAINING GRAPHENE QUANTUM DOT AND PREPARATION METHOD THEREFOR**

(30) Priority: 25.08.2020 KR 20200107307; 24.08.2021 KR 20210111342
(71) Applicant: Biographene Inc., Suwon-si, Gyeonggi-do 16229 (KR); Seoul National University R & DB Foundation, Seoul 08826 (KR)
(72) Inventor: HONG, Byung Hee, Suwon-si Gyeonggi-do 16420 (KR); JUNG, Young Jin, Suwon-si Gyeonggi-do 16226 (KR); PARK, Jong Bo, Seoul 08539 (KR)
(74) Representative: Nederlandsch Octrooibureau
(86) International application number: PCT/KR2021/011324
(87) International publication number: WO 2022/045748

(57) **Abstract**

The present invention relates to a method for preparing a carbon-13-containing graphene quantum dot available as an MRI contrast medium, a carbon-13-containing graphene quantum dot prepared by the method, and an MRI contrast medium comprising the carbon-13 graphene quantum dot.

## Description

### [Technical Field]

The present invention relates to an MRI contrast medium comprising a carbon-13 (¹³C)-containing graphene quantum dot, a preparation method thereof, and the like, and more particularly, to a method for preparing a carbon-13 containing graphene quantum dot usable as an MRI contrast medium, a carbon-13-containing graphene quantum dot prepared using the preparation method, and an MRI contrast medium comprising the graphene quantum dot.

### [Background Art]

Magnetic resonance imaging (MRI) for diagnosis is one of the safest and non-destructive imaging techniques capable of diagnosing diseases by observing the inside of the human body, and has the advantage of not involving exposure to radioactivity, unlike other diagnostic imaging techniques, while being sensitive to human tissue. Therefore, the application and utilization rate of MRI tends to be increasing day by day, and since 2019, medical insurance coverage for MRI has been expanding in Korea.

As an MRI contrast agent, a colloidal solution using a paramagnetic material such as gadolinium, manganese, and iron oxide is used, and in particular, commercially available gadolinium-based products are currently most widely used. However, since gadolinium, manganese and the like are highly toxic, they are used as chelates with organic materials to reduce their toxicity. However, this is still known to induce various side effects in the human body. For example, relatively mild symptoms such as itching, vomiting, nausea, and rashes and serious side effects such as renal failure, systemic fibrosis, anaphylactic shock, and cardiac arrest may occur in some cases, and may even lead to death (Korean Patent No. 10-2020870).

Accordingly, there have been attempts to develop contrast agents comprising fullerenes and carbon nanotubes using ¹³C, which is harmless to the human body and can be used as a contrast agent, but since fullerenes and carbon nanotubes do not dissolve well in water, there is a limitation in that they can only be used as contrast agents when they are combined with a substrate that dissolves well in water. Further, when fullerenes and carbon nanotubes undergo a dynamic nuclear polarization (DNP) process, since ¹³C MRI signals are intensified 10³-fold, it is difficult to substantially industrialize and efficiently use fullerenes and carbon nanotubes as contrast media because they need to undergo a dynamic nuclear polarization process.

Therefore, there is an urgent need for developing a novel contrast medium using ¹³C, which has low toxicity, is easily excreted from the body, is easily dissolved in an aqueous solution, and can remarkably increase sensitivity without undergoing a dynamic nuclear polarization process.

### [Disclosure]

### [Technical Problem]

The present invention has been devised to solve the problems in the related art as described above, and an object thereof is to provide a method for preparing a carbon-13-containing graphene quantum dot using microwaves, a magnetic resonance imaging contrast medium comprising a carbon-13-containing graphene quantum dot prepared by the preparation method as an active ingredient, and the like.

However, the technical problems which the present invention intends to solve are not limited to the technical problems which have been mentioned above, and other technical problems which have not been mentioned will clearly be understood by those with ordinary skill in the art to which the present invention pertains from the following description.

### [Technical Solution]

The present invention provides a method for preparing a magnetic resonance imaging contrast medium, the method comprising: a) preparing a saccharide solution by adding a carbon-13(¹³C)-containing saccharide to an aqueous solution; b) synthesizing a ¹³C-containing graphene quantum dot by treating the saccharide solution in Step a) with microwaves; and c) preparing a magnetic resonance imaging contrast medium comprising the ¹³C-containing graphene quantum dot.

In an exemplary embodiment of the present invention, the ¹³C-containing saccharide may be preferably any one or more selected from the group consisting of glucose, fructose, galactose, lactose, cellulose, glycogen, starch, arabinoxylan, sucrose, maltose and chitin, and is more preferably glucose, but is not limited thereto as long as the ¹³C-containing saccharide is a saccharide containing ¹³C.

In another exemplary embodiment of the present invention, the aqueous solution is preferably distilled water or an acidic aqueous solution, and the acidic aqueous solution means an aqueous solution prepared using lactic acid, acetic acid, formic acid, ascorbic acid, citric acid, oxalic acid, butyric acid, and the like, but is not limited unless the acidic aqueous solution is an organic solvent or an aqueous solution showing toxicity.

In still another exemplary embodiment of the present invention, the saccharide solution in Step a) may be prepared at a concentration of preferably 0.001 to 1 g/ml, more preferably 0.01 to 0.1 g/ml.

In yet another exemplary embodiment of the present invention, the saccharide solution may be preferably treated with microwaves in Step b) at a power consumption of 10 to 3000 watts (W) for 1 to 500 minutes, more preferably at a power consumption of 100 to 1000 watts for 10 to 300 minutes, even more preferably at a power consumption of 500 to 1000 watts for 20 to 100 minutes, and still more preferably at a power consumption of 700 to 900 watts for 20 to 50 minutes.

In yet another exemplary embodiment of the present invention, the ¹³C-containing graphene quantum dot may preferably have an average diameter of 0.1 to 100 nm, more preferably 0.1 to 10 nm.

In yet another exemplary embodiment of the present invention, the magnetic resonance imaging contrast medium is characterized by being accumulated in the reticuloendothelial system comprising the liver or spleen and used to obtain images thereof.

In yet another exemplary embodiment of the present invention, the magnetic resonance imaging contrast medium is characterized by being used to obtain images of blood vessels, lymph nodes, or angiogenesis of cancer cells, or atherosclerotic plaque.

In yet another exemplary embodiment of the present invention, the magnetic resonance imaging contrast medium is characterized by being a multi-imaging contrast medium added to magnetic resonance images and used to obtain images comprising Raman imaging, positron emission tomography (PET), or fluorescence imaging.

In yet another exemplary embodiment of the present invention, the magnetic resonance imaging contrast medium is characterized by being a T1 contrast medium.

In addition, the present invention provides a magnetic resonance imaging contrast medium comprising a ¹³C-containing graphene quantum dot as an active ingredient.

In an exemplary embodiment of the present invention, the ¹³C-containing graphene quantum dot is characterized by consisting of carbon and oxygen.

Furthermore, the present invention provides an image monitoring method comprising administering the magnetic resonance imaging contrast medium prepared by the method to a subject in need.

### [Advantageous Effects]

Since a method for preparing a magnetic resonance imaging contrast medium comprising the carbon-13-containing graphene quantum dot of the present invention as an active ingredient can easily prepare a carbon-13-containing graphene quantum dot by a one-step method within a short period of time within 1 hour and can prepare the carbon-13-containing graphene quantum dot without using a harmful material such as an organic solvent during the preparation process, no post-treatment process step for subsequent removal is required, and stability can be enhanced. Further, since the magnetic resonance imaging contrast medium of the present invention consists only of carbon, oxygen, and hydrogen, and thus has low toxicity and can be easily dispersed in an aqueous solution using a saccharide, the magnetic resonance imaging contrast medium can be easily excreted from the body when administered to the body, and it is expected to reduce the incidence of nephrogenic systemic fibrosis frequently occurring in patients with impaired renal function when the MRI contrast agent is used, so that the magnetic resonance imaging contrast medium of the present invention is expected to be stably used in various patients as an MRI contrast agent.

### [Description of Drawings]

FIG 1 is a set of views showing the synthesis mechanism and synthesis results of the carbon-13-containing graphene quantum dot according to an exemplary embodiment of the present invention.
FIG 2 is a view showing the results of analyzing the characteristics of the carbon-13-containing graphene quantum dot according to an exemplary embodiment of the present invention using Fourier transform infrared spectroscopy.
FIG 3 is a set of views showing the results of analyzing the characteristics of the carbon-13-containing graphene quantum dot according to an exemplary embodiment of the present invention using Raman spectroscopy.
FIG 4 is a set of views showing the results of analyzing the characteristics of the carbon-13-containing graphene quantum dot according to an exemplary embodiment of the present invention using x-ray photoelectron spectroscopy.
FIG 5 is a set of views showing the results of analyzing the characteristics of the carbon-13-containing graphene quantum dot according to an exemplary embodiment of the present invention using a transmission electron microscope.
FIG 6 is a set of views showing the results of confirming the imaging effect of the carbon-13-containing graphene quantum dot according to an exemplary embodiment of the present invention through a phantom imaging test.

### [Modes of the Invention]

As a result of conducting intensive studies to develop a magnetic resonance imaging contrast medium which has low toxicity *in vivo* and has excellent solubility, the present inventors can easily prepare a carbon-13-containing graphene quantum dot with increased solubility and reduced toxicity within a short period of time by treating saccharides with microwaves, and developed a magnetic resonance imaging contrast medium comprising a carbon-13-containing graphene quantum dot prepared by the method of the present invention as an active ingredient through this.

As used herein, "grapheme" means that a plurality of carbon atoms are covalently linked to each other to form a polycyclic aromatic molecule, and the covalently linked carbon atoms form a 6-membered ring as a basic repeating unit, but it is also possible to further comprise a 5-membered ring and/or a 7-membered ring.

As used herein, "graphene quantum dots (GQDs)" refer to nano-sized graphene fragments. Graphene quantum dots are known to have almost no biotoxicity and are easily excreted from the body when administered to the body. The graphene quantum dot of the present invention may be present in a solid or liquid form. A solvate may comprise a non-aqueous solvent such as ethanol, isopropanol, DMSO, acetic acid, ethanolamine, and ethyl acetate, or may comprise water as a solvent which is incorporated into the crystalline lattice. A solvate, in which water is the solvent incorporated into the crystalline lattice, is typically referred to as a "hydrate". The present invention comprises all such solvates.

In the present specification, the carbon-13(¹³C)-containing graphene quantum dots may have an average diameter of, for example, 0.1 to 100 nm, 0.1 to 90 nm, 0.1 to 80 nm, 0.1 to 70 nm, 0.1 to 60 nm, 0.1 to 50 nm, 0.1 to 40 nm, 0.1 to 30 nm, 0.1 to 20 nm, 0.1 to 10 nm, 0.1 to 5 nm, 0.1 to 3 nm, 1 to 100nm, 1 to 90 nm, 1 to 80 nm, 1 to 70 nm, 1 to 60 nm, 1 to 50 nm, 1 to 40 nm, 1 to 30 nm, 1 to 20 nm, 1 to 10 nm, 1 to 5 nm, 1 to 3 nm, 2 to 100 nm, 2 to 90 nm, 2 to 80 nm, 2 to 70 nm, 2 to 60 nm, 2 to 50 nm, 2 to 40 nm, 2 to 30 nm, 2 to 20 nm, 2 to 10 nm, 2 to 5 nm, and 2 to 3 nm, and may have an average diameter of 2 to 5 nm according to an exemplary embodiment of the present invention, but is not limited thereto. In addition, the carbon-13(¹³C)-containing graphene quantum dot may have the following structural features: 1) comprises a functional group comprising oxygen, such as a hydroxyl group (-OH) and a carboxyl group (-COOH), 2) exhibits a G-peak in the Raman spectrum, 3) consists of carbon-carbon single bonds and carbon-carbon double bonds, and carbon-oxygen bonds as a material consisting only of carbon and oxygen, and 4) has crystallinity inherent to graphene, but is not limited thereto.

As used herein, "saccharides" comprise linear or cyclic monosaccharides, oligosaccharides or polysaccharides. Oligosaccharides refer to saccharides with two or more monosaccharide residues. Saccharides may be monosaccharides or polysaccharides, and as some examples of saccharides, the monosaccharide may be glucose, fructose, galactose, mannose, xylose, ribose, arabinose, lyxose, sucrose, maltose, and the like, and the polysaccharide may be lactose, cellulose, glycogen, starch, chitin, an oligosaccharide, dextran, chitosan, callose, laminarin, chrysolaminarin, xylan, arabinoxylan, mannan, fucoidan, and galactomannan, and may be glucose according to an exemplary embodiment of the present invention, but is not limited thereto.

As used herein, "microwaves" refer to electromagnetic waves having a wavelength and frequency between radio waves and infrared rays, usually refers to electromagnetic radiation with a wavelength between 1 mm and 10 cm, and is a generic term for electromagnetic waves in a frequency band of about 300 MHz to about 300 GHz. In terms of wavelength in vacuum, microwaves are electromagnetic waves with a wavelength of about 1 m to 1 mm. This microwave comprises millimeter waves of about 30 GHz to 300 GHz. Waves having a short wavelength, such as microwaves, have similar properties to light waves, and thus have rectilinear propagation, reflection, and absorption properties. Accordingly, such waves may be used to heat foods through microwave dielectric heating, defrost frozen foods, simply sterilize foods, and sterilize pests in foods as well as being widely used for communication (transmission of electric waves). A magnetron is usually used to generate microwaves.

In the present specification, the "magnetic resonance imaging contrast agent" may comprise those in which the proportion of ¹³C contained in the carbon-13 (¹³C)-containing graphene quantum dot is about 5% to about 100% of the total carbon atoms, but is not limited thereto. For example, the proportion of ¹³C contained in the carbon-13(¹³C)-containing graphene quantum dot may be about 5% to about 100%, about 10 % to about 100%, about 20% to about 100%, about 30% to about 100%, about 40% to about 100%, about 50% to about 100%, about 60% to about 100%, about 70% to about 100%, about 80% to about 100%, about 90% to about 100%, about 10% to about 90%, about 10% to about 80%, about 10% to about 70%, about 10% to about 60%, about 10% to about 50%, about 10% to about 40%, about 10% to about 30%, or about 10% to about 20%, but is not limited thereto. Furthermore, the magnetic resonance signal detection sensitivity may increase as the ¹³C content proportion of the carbon-13(¹³C)-containing graphene quantum dots increases, but is not limited thereto.

The magnetic resonance imaging contrast medium may be a multi-imaging contrast medium added to magnetic resonance images and used to obtain images comprising Raman imaging, positron emission tomography (PET), or fluorescence imaging, but is not limited thereto. A multi-imaging contrast agent that enables multi-imaging with other imaging devices based on magnetic resonance imaging enables quick and accurate diagnosis of a desired disease and accurate recognition of the disease before entering the treatment stage. For example, various types of information about lesions may be obtained through multi-imaging by simultaneously performing tomographic imaging by MRI and molecular imaging by PET, and it is possible to comprise those showing the results that allow more reliable recognition of the lesion state by compensating for each other's disadvantages.

MRI contrast agents are generally classified into two types, among them, T1 contrast agents are also called positive contrast agents because they exhibit the effect of brightening the corresponding area in the MRI image, and T2 contrast agents darken the corresponding area through magnetic field disturbance with MRI equipment, and thus are called negative contrast agents. In general, it is known that T2 contrast agents have the disadvantage of causing confusion in the presence of bleeding, calcification, thrombi, metal deposition, and the like, and thus are less competitive.

In the present invention, the magnetic resonance imaging (MRI) contrast agent may comprise a bioactive material is bonded to the outer surface of a carbon-13(¹³C)-containing graphene quantum dot, but is not limited thereto. For example, the bioactive material may comprise an antibody that recognizes and selectively binds to a specific antigen by *in vivo* immune activation; a monoclonal antibody or a polyclonal antibody produced based thereon; a variable region or constant region of an antibody; a chimeric antibody that is partially or entirely modified; a humanized antibody; a nucleic acid such as DNA or RNA capable of complementary binding to DNA or RNA having a specific base sequence; a targeting material comprising non-biological chemical materials and the like capable of binding to a specific chemical functional group under certain conditions through hydrogen bonds and the like; various pharmacologically active materials that have therapeutic, preventive, or symptom alleviation effects on various diseases; a toxic active material such as a gene or a toxic protein that induces cell death; a chemical sensitive to electromagnetic waves, magnetic fields, electric fields, light, or heat; a fluorescent material that generates fluorescence; a bioactive material such as an isotope that generates radiation; or a glucose tracer, and the like.

Furthermore, the bioactive material comprises all bioactive materials already known in the related art, and more specifically, comprises all bioactive materials that can be bonded by methods known in the related art. For example, the carbon-13(¹³C)-containing graphene quantum dot may be bonded to an active ingredient that is pharmaceutically active; or an active ingredient that is sensitive to radiation, electric fields, magnetic fields, various electromagnetic waves, heat or light and is capable of diagnosing and/or treating tumors, specific proteins, and the like, but is not limited thereto.

Further, the carbon-13(¹³C)-containing graphene quantum dot bonded to the bioactive material may be used for diagnosing and/or treating various diseases associated with various tumors such as gastric cancer, lung cancer, breast cancer, liver cancer, laryngeal cancer, cervical cancer, ovarian cancer, bronchial cancer, nasopharyngeal cancer, pancreatic cancer, bladder cancer or colon cancer, and various diseases associated with a specific protein such as Alzheimer's disease, Parkinson's disease, or Mad cow disease, but is not limited thereto.

In the present invention, the bioactive material may comprise: a bioactive material selected from the group consisting of a targeting material selected from the group consisting of a protein, RNA, DNA, an antibody, and a combination thereof, which selectively bind to a target material *in vivo;* an apoptosis-inducing gene or a toxic protein; a fluorescent material; an isotope: a material sensitive to light, electromagnetic waves, radiation, or heat; a pharmacologically active material; and a combination thereof.

As used herein, "a subject in need" refers to a subject to which the composition of the present invention may be administered, and the subject is not limited.

Hereinafter, preferred examples for helping with understanding of the present invention will be suggested. However, the following examples are provided only so that the present invention may be more easily understood, and the content of the present invention is not limited by the following examples.

### [Examples]

### Example 1. Preparation of carbon-13-containing graphene quantum dot

### 1.1. Preparation of carbon-13-containing graphene quantum dot

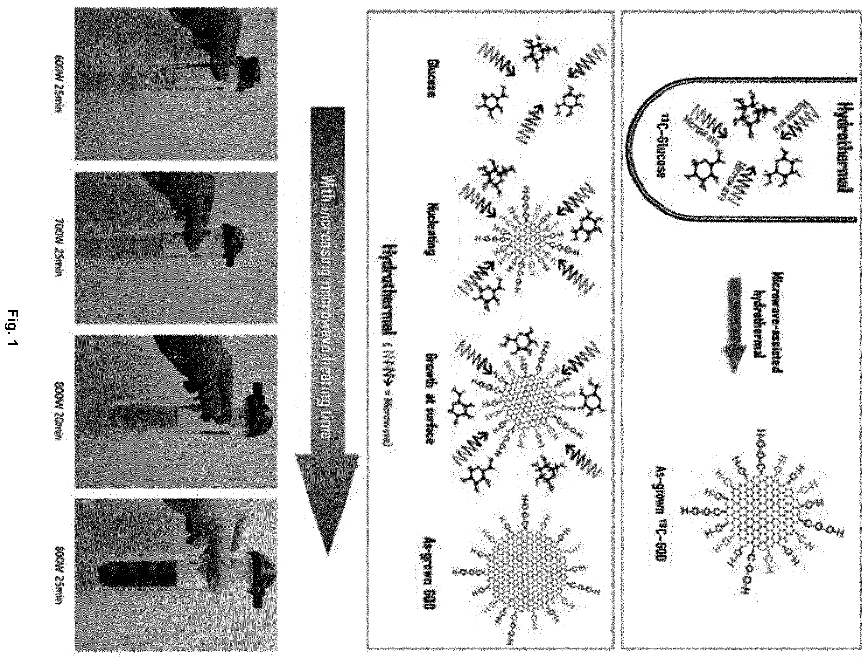

To prepare carbon-13(¹³C)-containing graphene quantum dots (GODs) that can be dissolved in an aqueous solution, a carbon-13-containing glucose solution (¹³C glucose solution) was added to distilled water at a concentration of 0.001 to 0.1 g/ml, and microwave treatment was performed in a reaction vessel for water treatment. The solution was treated with microwaves at a power consumption of 100 to 1000 watts (W) for 10 to 300 minutes. A hydrothermal reaction was induced by the microwave treatment to synthesize carbon-13-containing graphene quantum dots in a bottom-up fashion. The synthesis mechanism and synthesis results of carbon-13-containing graphene quantum dots are shown in FIG 1.

As shown in FIG 1, a water condensation reaction was induced from glucose through microwave treatment to prepare carbon-13-containing graphene quantum dots, and it could be confirmed through the color of the aqueous solution that the synthesis rate of carbon-13-containing graphene quantum dots was increased by increasing the microwave intensity or reaction time.

### 1.2. Characterization of carbon-13-containing graphene quantum dots

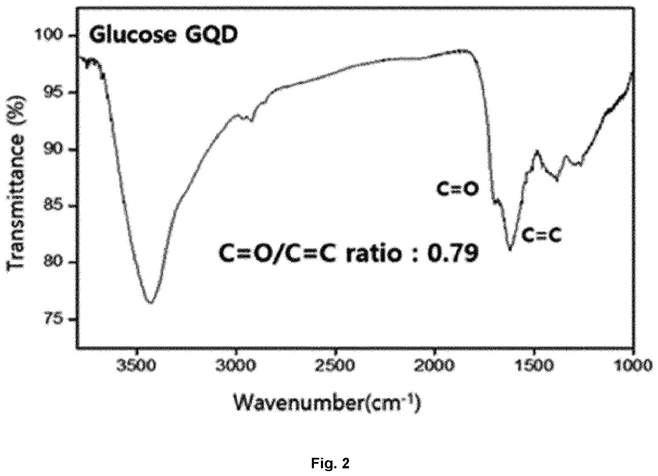

The characteristics of carbon-13-containing graphene quantum dots prepared in the same manner as in Example 1.1 were analyzed. First, chemical properties were analyzed using Fourier transform infrared spectroscopy (FT-IR). The results are shown in FIG 2.

As shown in FIG 2, it was confirmed that a hydroxyl group (-OH), a carboxyl group (-COOH), and the like, which are oxygen-containing functional groups, were present in the carbon-13-containing graphene quantum dot, and it was confirmed that the intensity ratio of the C=O/C=C bond peak was about 0.79.

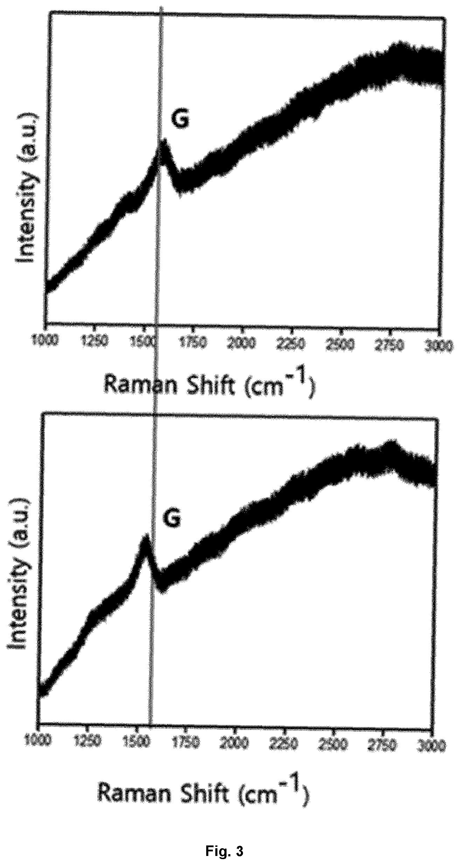

In addition, Raman spectra were confirmed using Raman spectroscopy (CRM200, Witech). The results are shown in FIG 3.

As shown in FIG 3, as a result of comparing a carbon-12-containing graphene quantum dot (upper graph) and a carbon-13-containing graphene quantum dot (lower graph), it was confirmed that a peak shift appeared due to changes in the molecular vibration frequency and a G-peak appeared in both graphene quantum dots. Through the above results, it was confirmed that carbon-13-containing graphene quantum dots were successfully prepared by graphitization from glucose.

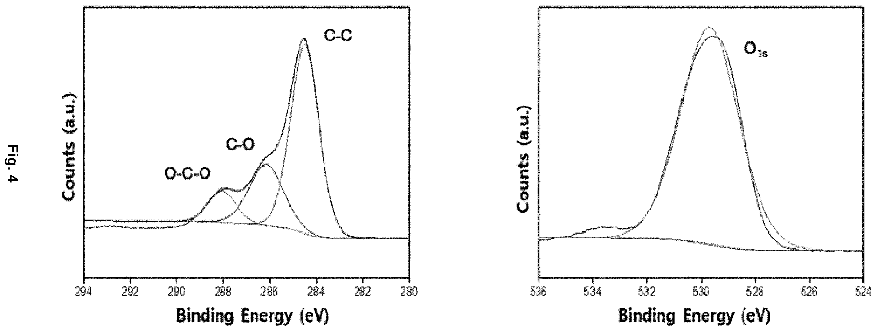

Next, X-ray photoelectron spectroscopy (XPS) was used to analyze the characteristics of the carbon-13-containing graphene quantum dot. The results are shown in FIG 4.

As shown in FIG 4, it was confirmed that the carbon-13-containing graphene quantum dot consists of carbon-carbon single bonds, carbon-carbon double bonds, and carbon-oxygen bonds as a material consisting only of carbon and oxygen.

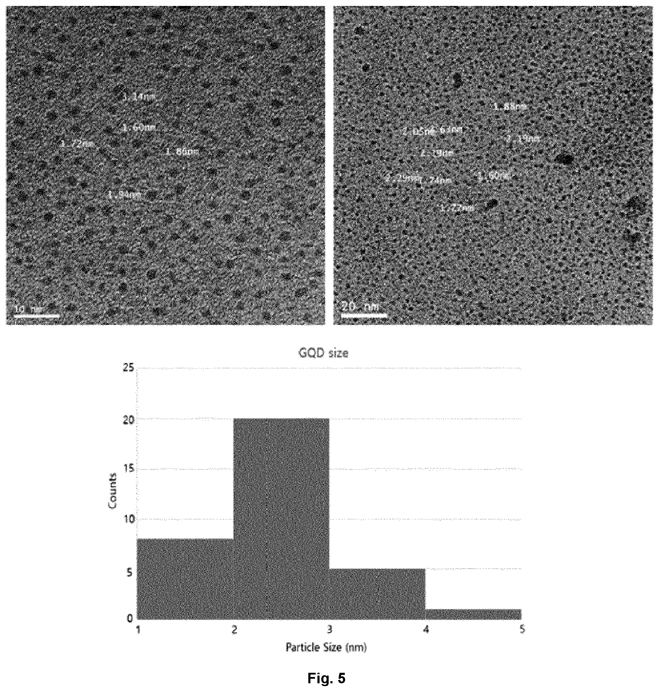

Further, carbon-13 graphene quantum dots were confirmed using a transmission electron microscopy (TEM). The results are shown in FIG 5.

As shown in FIG 5, it was confirmed that the carbon-13-containing graphene quantum dot has a uniform circular morphology with a diameter of about 2 to 5 nm.

### Example 2. Confirmation of contrast effect of carbon-13-containing graphene quantum dots

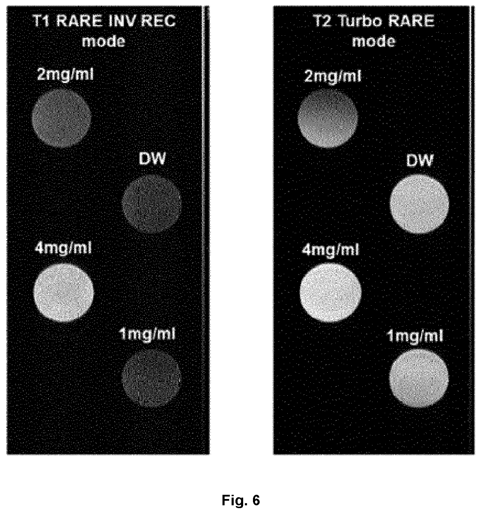

In order to confirm the imaging effect of carbon-13-containing graphene quantum dots prepared in the same manner as in Example 1.1, a phantom imaging test was performed using carbon-13-containing graphene quantum dots with different concentrations in various measurement modes. T₁ measurement was confirmed using inversion recovery according to various T₁ times (inversion times), and T₂ measurement was measured by applying the Carr Purcell Meiboom Gill (CPMG) pulse sequence for various spin-echo measurements. The results are shown in FIG 6.

As shown in FIG 6, it was confirmed that, in the T1 RARE INV REC mode, as the concentration of carbon-13-containing graphene quantum dots contained in the solution increased, the contrast effect appeared stronger. However, it was confirmed that the T2 Turbo RARE mode did not show a significant difference according to concentration. Through the above results, it could be confirmed that the carbon-13-containing graphene quantum dot of the present invention can be used as a T1 MRI contrast agent.

Through the above results, it was confirmed that the carbon-13-containing graphene quantum dots were normally prepared by the method for preparing carbon-13 containing graphene quantum dots using microwaves of the present invention, and it was confirmed that the carbon-13-containing graphene quantum dot prepared by the method of the present invention can be used as a T1 MRI contrast agent. In addition, it was confirmed that compared to the existing thermo-oxidative cutting method or bottom-up method, the size of the carbon-13-containing graphene quantum dots prepared by the microwave-based preparation method of the present invention was uniform and impurities were reduced. Furthermore, by synthesizing the carbon-13-containing graphene quantum dots using microwaves, it is easy to adjust the proportion of a functional group such as a carboxyl group according to the type of reactant and solvent. Further, since carbon-13-containing graphene quantum dots can be easily prepared within a short period of time by a one-step method and can be prepared without using a harmful material such as an organic solvent during the preparation process, no post-treatment process step for removal is required, as a result, the mass production and industrialization of carbon-13-containing graphene quantum dots can be enabled because production costs can be remarkably reduced. In addition, since the carbon-13-containing graphene quantum dot of the present invention consists only of carbon and oxygen, and not only has low toxicity, but also can be easily dispersed in an aqueous solution using a saccharide, the carbon-13-containing graphene quantum dot is expected to be used effectively as an MRI contrast medium because it can be easily dispersed in an aqueous solution.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

Since a method for preparing a magnetic resonance imaging contrast medium using microwaves of the present invention can easily prepare a carbon-13-containing graphene quantum dot by a one-step method within a short period of time and can prepare the carbon-13-containing graphene quantum dot without using a harmful material such as an organic solvent during the preparation process, no post-treatment process step for subsequent removal is required, thereby remarkably reducing production costs. Therefore, the mass production and industrialization of a magnetic resonance imaging contrast agent comprising a carbon-13-containing graphene quantum dot as active ingredient can be enabled.

## Claims

1. A method for preparing a magnetic resonance contrast medium, the method comprising: a) preparing a saccharide solution by adding a carbon-13(¹³C)-containing saccharide to an aqueous solution;
b) synthesizing a ¹³C-containing graphene quantum dot by treating the saccharide solution in Step a) with microwaves; and
c) preparing a magnetic resonance imaging contrast medium comprising the ¹³C-containing graphene quantum dot.

2. The method of claim 1, wherein the ¹³C-containing saccharide is any one or more selected from the group consisting of glucose, fructose, galactose, lactose, cellulose, glycogen, starch, arabinoxylan, sucrose, maltose and chitin.

3. The method of claim 1, wherein the aqueous solution is distilled water or an acidic aqueous solution.

4. The method of claim 1, wherein the saccharide solution in Step a) is prepared at a concentration of 0.001 to 1 g/ml.

5. The method of claim 1, wherein the saccharide solution is treated with microwaves in Step b) at a power consumption of 10 to 3000 watts (W) for 1 to 500 minutes.

6. The method of claim 1, wherein the ¹³C-containing graphene quantum dot has an average diameter of 0.1 to 100 nm.

7. The method of claim 1, wherein the magnetic resonance imaging contrast medium is accumulated in the reticuloendothelial system comprising the liver or spleen and used to obtain images thereof.

8. The method of claim 1, wherein the magnetic resonance imaging contrast medium is used to obtain images of blood vessels, lymph nodes, or angiogenesis of cancer cells, or atherosclerotic plaque.

9. The method of claim 1, wherein the magnetic resonance imaging contrast medium is a multi-imaging contrast medium added to magnetic resonance images and used to obtain images comprising Raman imaging, positron emission tomography (PET), or fluorescence imaging.

10. The method of claim 1, wherein the magnetic resonance imaging contrast medium is a T1 contrast medium.

11. A magnetic resonance imaging contrast medium comprising a ¹³C-containing graphene quantum dot prepared by the method of any one of claims 1 to 10 as an active ingredient.

12. The magnetic resonance imaging contrast medium of claim 11, wherein the ¹³C-containing graphene quantum dot consists of carbon and oxygen.

13. An image monitoring method comprising administering the magnetic resonance imaging contrast medium of claim 11 to a subject in need.
